# EUROPEAN PATENT APPLICATION

(11) **EP 2 072 048 A1**
(43) Date of publication of application: **24.06.2009**
(21) Application number: 09075115.7
(22) Date of filing: 13.01.2006
(51) Int. Cl.: A61K 31/202, A61P 3/04, A61P 37/04

(54) **Use of pinolenic acid for the treatment of obesity**

(30) Priority: 31.01.2005 EP 05250517; 14.07.2005 GB 0514463
(62) Divisional of application: 06250162.2
(71) Applicant: Lipid Nutrition B.V., 1521 AZ Wormerveer (NL)
(72) Inventor: Stam, Wiro Bartholomeus, 1521 AZ Wormerveer (NL); Einerhand, Alexandra Wilhelmina Carla, 1521 AZ Wormerveer (NL); Schmid, Ulrike, 1521 AZ Wormerveer (NL); Heimerikx, Jos, 1521 AZ Wornerveer (NL); Gambelli, Luisa, 1521 AZ Wormerveer (NL)
(74) Representative: Stevens, Ian Edward

(57) **Abstract**

Pinolenic acid or a derivative thereof can be used for weight management. The pinolenic acid may be used in the form of a pharmaceutical composition.

## Description

This invention relates to compositions that may be used for weight management, for controlling food intake and appetite, and for controlling and/or reducing body weight. The compositions contain pinolenic acid or a derivative thereof.

Obesity is becoming increasingly prevalent in individuals in developed societies. Generally, an overweight condition and obesity result from an imbalance in energy intake and utilisation. The cause of energy imbalance for each individual may be due to a combination of several factors and stems from a myriad of both environmental and genetic determinants. Obesity may be a contributing factor in the increased incidence of various diseases including coronary artery disease, hypertension, stroke, diabetes and certain cancers.

Weight reduction is often recommended as the first course of action for patients suffering from these obesity-related diseases. In an attempt to control total body weight, an individual may undertake weight management, the objectives of which may differ depending on the needs of the individual. For example, whereas obese or overweight individuals may wish to lose body weight, other individuals may wish to maintain a body weight at a substantially constant level. Even once a person has lost body weight, weight management is often required to prevent that person regaining the body weight previously lost. The most effective weight loss programmes typically include a reduced calorie diet and/or increased energy expenditure. Over time, many people undertaking weight management experience increased hunger levels and/or cravings for high sugar foods. This can lead individuals to stray from their weight management regime. There is, therefore, a need to develop new and effective ways to support weight management and to help individuals continue with their weight management regime. It is an object of the present invention to provide a new means for providing this support.

Pinolenic acid (i.e., 5, 9, 12 C18:3 fatty acid, a faity acid with 18 C atoms having three double bonds in the positions 5, 9 and 12) is present in, for example, pine nut oil and fractions thereof (J Am Oil Chem Soc 1998, 75, p.45-50). It is known that pine nut oil, and thus pinolenic acid, can be applied in food products, see for example, FR-A-2756465 wherein the use of a concentrate with 15% pinolenic acid in food additives is disclosed. The presence of pinolenic acid is described as providing a hypolipemic effect to the composition.

WO 98/43513 discloses that nail files can be coated with pinolenic acid and that this inhibits the occurrence of infections upon use of the files.

According to JP-A-61238729, pine nut oil can be used as an anticholesteric agent. Other documents wherein health effects of pinolenic acid are disclosed include JP-A-61058536, wherein a very generic activity beneficial for human health is disclosed. This health activity is not disclosed in further detail. Sugano, Brit J. of Nutr 72 (1994) 775-783, discloses a number of health effects of diets containing pinolenic acid. The health effects mentioned are hypocholesterolaemic effects, effects on ADP-induced platelet aggregation, on aortic prostacylic production and on blood pressure. EP-A-1098552 describes the use of pinolenic acid as an antiinflammatory agent.

Matsuo et al, Prostaglandins, Leukotrienes and Essential Fatty Acids, (1996), 55(4), 223-229 describes the effects of y-linolenic and pinolenic acid on immune parameters of Brown-Norway rats.

However, none of the prior art documents indicate that pinolenic acid or a derivative thereof could be used to treat or prevent obesity and/or for weight management.

US 6,429,190, US 2002/0119948 and US 2002/0119915 describe compositions for extending satiety comprising a calcium source, protein and a C12 to C18 fatty acid. Oleic acid is the fatty acid described in the documents.

WO 02/008159 discloses pharmaceutically active uridine esters, and combinations comprising their constituent acid and uridine compound, and their use in a wide variety of medical applications. There is no disclosure of the use of free fatty acids alone nor is any example given of the treatment of obesity.

EP-A-1504778, published on 9 February 2005, describes an implantable pump for the treatment of obesity. The pump may comprise a fatty acid but there is no disclosure of pinolenic acid.

CN-A-1377673 relates to the use of a pine nut oil for treating cardiac and cerebral vascular diseases and adiposity caused by hyperlipemia as well as diabetes caused by hyperglycemia.

The present invention provides the use of pinolenic acid or a derivative thereof in the manufacture of a composition for weight management by reducing the feeling of hunger and/or increasing satiety. The invention also provides the use of pinolenic acid or a derivative thereof for reducing the feeling of hunger and/or increasing satiety. Thus, compositions of the invention are suitable for treating or preventing obesity and/or for weight management and comprise pinolenic acid or a derivative thereof. Derivatives of pinolenic acid, which can be used in the present invention, include salts of pinolenic acid and alkyl esters. Other derivatives of pinolenic acid which can be used in the invention are isomers of pinolenic acid such as, for example, geometric isomers (having one or more trans double bonds; the double bonds in pinolenic acid are all cis) and/or compounds having 18 carbon atoms and three double bonds with one or more of the three double bonds at a different position in the alkyl chain compared to pinolenic acid, including, for example, gamma linolenic acid, alpha linolenic acid, punicic acid, eleostearic acid, and their salts and alkyl esters.

The invention also covers, therefore: the use of alpha-linolenic acid or a derivative thereof for weight management by reducing the feeling of hunger and/or increasing satiety; the use of gamma-linolenic acid or a derivative thereof for weight management by reducing the feeling of hunger and/or increasing satiety; the use of punicic acid or a derivative thereof for weight management by reducing the feeling of hunger and/or increasing satiety; and the use of eleostearic acid (also termed alpha-eleostearic acid) or a derivative thereof for weight management by reducing the feeling of hunger and/or increasing satiety.

The invention also contemplates pinolenic acid or a derivative thereof for treating or preventing obesity and/or for weight management. The pinolenic acid or derivative thereof may be used alone or may be one component of a composition which comprises other edible and/or pharmaceutically acceptable components. Preferably, the composition further comprises from 10 to 60 wt% of linoleic acid and/or from 5 to 52 wt% oleic acid, either as free acids or glycerides (e.g., mono-, di- or tri- glycerides). Additionally or alternatively, the composition may comprise from 0.5 to 5 wt% of taxoleic acid.

It has surprisingly been found that the fatty acids of pine nut oil, and thus pinolenic acid, can be used to stimulate the release of cholecystokinin (CCK). CCK is a peptide released following the consumption of food. When food is consumed, CCK releasing protein (CCKRP) is released in the small intestine. CCKRP stimulates CCK release from intestinal cells. The release of CCK generates the behavioural symptoms associated with satiety. Additionally, increased CCK levels can increase IgA levels in the gut that can increase mucosal immunity. Increased levels of IgA in the intestinal tract may offer increased protection against invading microorganisms. Therefore, the invention also contemplates a method of increasing immunity (e.g., mucosal immunity) comprising the administration of pinolenic acid or a derivative thereof, pinolenic acid or a derivative thereof for increasing immunity (e.g., mucosal immunity) and the use of pinolenic acid or a derivative thereof in the manufacture of a composition for increasing immunity (e.g., mucosal immunity). Increasing immunity may be used in the treatment and/or prevention of infections, such as bacterial or viral infections.

In broad terms, the invention relates to the use of straight chain carboxylic acids (and their derivatives such as salts and esters) having 18 carbon atoms and three double bonds for weight management and/or for treating or preventing obesity. Weight management may comprise reducing the feeling of hunger and/or increasing satiety.

The compositions of the invention may be in any suitable form such as a food supplement, a pharmaceutical composition or a food composition. The term composition means that the pinolenic acid or derivative thereof may be present with one or more other components (e.g., as are present in pine nut oil). The one or more other components may be present in admixture with the pinolenic acid or derivative or they may form part of the packaging of the product (e.g., the capsule in which the pinolenic acid or derivative is encapsulated).

The compositions of the invention typically comprise from 0.3 to 100 wt%, preferably 5 to 80 wt%, most preferably 10 to 50 wt% (such as 10 to 30 wt% or 20 to 30 wt%) of linolenic acid or a derivative thereof. The skilled person will appreciate that the percentage of pinolenic acid or a derivative thereof in a composition of the invention will depend on the nature of the composition. For example, the pinolenic acid or derivative thereof is likely to represent a higher percentage of the total weight of a pharmaceutical composition or a food supplement than a food composition.

When the composition of the invention is in the form of a food supplement or a pharmaceutical product, the pinolenic acid or derivative thereof or a blend containing one of these compounds may be in encapsulated form in a food grade encapsulating material. Suitable encapsulating materials are well known in the art and include, for example, gelatin and glycerol.

The pinolenic acid used in the present invention may be in the form of a free fatty acid, a derivative of pinolenic acid or mixtures thereof, including mixtures of different derivatives. Derivatives are non-toxic and edible and preferably include, for example, salts and esters. Other derivatives of pinolenic acid which can be used in the invention are isomers of pinolenic acid such as, for example, geometric isomers (having one or more trans double bonds; the double bonds in pinolenic acid are all cis) and/or compounds having 18 carbon atoms and three double bonds with one or more of the three double bonds at a different position in the alkyl chain compared to pinolenic acid, including, for example, gamma linolenic acid, alpha linolenic acid, punicic acid, eleostearic acid, and their salts and alkyl esters. Suitable salts include salts with food grade cations such as sodium salts. Suitable esters include alkyl esters having from one to six carbon atoms. Preferred esters are mono-, di- and tri- glycerides.

Preferably, compositions of the invention are free of (or substantially free of, i.e., containing less than 0.1 mg of) uridine esters, and/or nucleosides and/or nucleotides selected from the group consisting of uridine, deoxyuridine, uridine monophosphate, deoxyuridine monophosphate and/or pharmaceutically acceptable salts thereof.

A suitable source for the pinolenic acid used in the present invention is pine nut oil or concentrates thereof. For example, glycerides of pinolenic acid can be obtained from pine nut oil or concentrates thereof. Preferably, an oil or concentrate with a content of pinolenic acid or a derivative thereof of more than 15 wt% or more than 28 wt% is used.

The compositions of the invention may comprise one or more other fatty acids (i.e., straight chain carboxylic acids having from 12 to 24 carbon atoms). Examples of other fatty acids suitable for use in the present invention include linoleic acid, oleic acid, taxoleic, juniperonic, sciadonic, saturated fatty acids, conjugated linoleic acid (optionally as an enriched isomer mixture) and EPA (eicosapentaenoic) and DHA (docosahexaenoic) (optionally as an enriched isomer mixture of EPA or DHA). Enrichment involves the alteration of the isomer mixture normally present (for example in a natural product), such as an alteration in the relative amounts of different geometrical isomers. In these compositions, the other fatty acid or each of the other fatty acids can independently be present as a free fatty acid or as a derivative thereof (including a mono-, di- or triglyceride and salts), or as a mixture thereof.

The pinolenic acid or derivative thereof are optionally blended with these additional fatty acids or glycerides before being used to prepare a composition according to the present invention. When the compositions of the invention contain one or more fatty acids and/or glycerides in addition to the pinolenic acid or derivative thereof, the additional fatty acid(s) and/or glycerides are preferably selected from liquid oils, such as soybean oil, sunflower oil, rape seed oil and cotton seed oil; cocoa butter and cocoa butter equivalents; palm oil and fractions thereof; enzymically made fats; fish oils and fractions thereof; conjugated linoleic acid and enriched isomer mixtures; gamma linolenic acid and enriched mixtures thereof; hardened liquid oils; and mixtures thereof.

Blends containing one or more additional fatty acids or glycerides preferably display solid fat contents measured by NMR-pulse on non stabilised fats of:
N20 = 1 - 80, preferably 5 - 45
N35 less than 20, preferably less than 10, most preferably less than 5.
These values were obtained by melting the fat at 80°C, cooling to 0°C and holding the fat at 0°C for 30 minutes, whereupon the fat was heated to the measurement temperature N and held at that temperature for 30 minutes before measuring the N value.

Blends of fatty acids that are used to produce the compositions of the invention preferably comprise from 1.5 to 60 wt%, more preferably from 28 to 60 wt% of pinolenic acid, from 10 to 60 wt% of linoleic acid and from 5 to 52 wt% of oleic acid, for example 25 to 85 wt% (linoleic plus oleic acid), from 0 to 70 wt%, for example 25 to 65 wt% (trans plus saturated fatty acid). The trans fatty acid content is preferably less than 10 wt%. An example of a suitable blend is one in which the trans plus saturated fatty acid content is less than 10 wt%.

Alternatively, the compositions of the invention may be free or substantially free of fatty acids other than pinolenic acid (i.e., may contain less than 1 % by weight, more preferably less than 0.1 % by weight, such as less than 0.01 % by weight of other C12 to C24 fatty acids).

The compositions of the invention may comprise calcium and/or magnesium sources. Additionally or alternatively, the compositions may comprise protein (including protein hydrolysates). The combination of pinolenic acid and calcium and/or magnesium and/or protein in the compositions of the present invention may increase the release of CCK from the intestinal cells.

In another embodiment, the compositions of the invention may be free or substantially free of calcium ions and/or protein (i.e., each of these components is present in the compositions in an amount of less than 1 % by weight, more preferably less than 0.1 % by weight, such as less than 0.01 % by weight).

Typically, compositions of the invention contain pinolenic acid and/or a derivative thereof as the sole active component.

The compositions of the present invention can be used to help manage body weight, for example to help maintain body weight at a substantially constant level or to help reduce body weight. In other words, use of the compositions can assist in slimming the body, for example by helping to induce fat loss.

The use of the compositions of the invention can help to reduce calorie intake. This can help maintain body weight at a substantially constant level and/or can help reduce body weight and/or help slimming. Reduction in body weight can increase energy levels.

The use of the compositions of the invention can reduce the feeling of hunger and/or increase satiety and/or reduce the desire for high calorie foods, for example by allowing less room in the stomach for high calorie foods. In particular, the use of the compositions of the invention can enhance and/or extend satiety or fullness prior to during and/or after a meal.

The use of the compositions of the invention can reduce the feeling of hunger during dieting and therefore increase the success rate of a diet.

Consumption of the composition of the invention can stimulate the release of CCK and/or IgA.

The use of the compositions of the invention can help to reduce appetite, for example by increasing satiation and a feeling of satiety and/or fullness.

The present invention also provides a method of treating or preventing obesity. In this method an effective amount of a composition as described above is administered to a mammal, for example a human. The present invention also provides a method of weight management. In this method an effective amount of a composition as described above is administered to a mammal, for example a human. Said administering need not be carried out by a physician or under medical supervision and can simply involve the mammal ingesting the composition e.g., in the form of a foodstuff or food supplement. Preferably, the compositions of the invention are administered (or taken) 2 hours to 3 minutes, more preferably 1 hour to 15 minutes and even more preferably 35 to 25 minutes before eating a meal.

The invention is applicable to mammals, preferably humans. Other mammals that may benefit from the compositions of the invention include pets (for example, dogs, cats, horses, rabbits, hamsters and guinea pigs) and farm animals (for example, cattle, sheep and pigs). Dogs and cats are particularly preferred.

When producing a food product, the pinolenic acid or derivative thereof can first be blended with structuring components for use in food products. However, this is not essential. These blends can, for example, be applied beneficially in food products as healthy fat compositions.

Pine nut oil can be used in the compositions of the invention. However, as pine nut oil can contain up to about 26 wt% of pinolenic acid, it would be advantageous (in particular for use in food supplements and to enable dosage forms of a smaller size) if concentrates could be obtained with higher levels of pinolenic acid. Concentrates of pinolenic acid or a derivative thereof to be used in the present invention can be prepared by any suitable process. A suitable process is described in EP-A-1088552.

In one suitable process, an enzymic hydrolysis or glycerolysis is performed using an enzyme that can discriminate between fatty acids with a delta 5 double bond and other fatty acids. This process comprises:
i) reacting a glyceride material containing at least 2 wt % of fatty acid with cis⁵ double bond with water or glycerol in the presence of an enzyme capable of discriminating between fatty acids containing a delta 5 double bond and other fatty acids;
ii) splitting the reaction mixture into a partial glyceride rich component and a fatty acid rich component;
iii) optionally converting the partial glycerides of step ii) to free fatty acids in the presence of a suitable enzyme
iv) optionally converting the fatty acid rich component of step ii) to triglycerides by reaction with glycerol in the presence of a suitable catalyst such as a suitable enzyme;
v) optionally splitting the partial glyceride rich material of step ii) into components that are a) rich in monoglycerides, b) rich in diglycerides and c) rich in triglycerides and then optionally converting the partial glycerides a) and b) into triglycerides by reaction with fatty acids in the presence of a suitable enzyme.

It is preferred to use a glyceride material with a pinolenic acid content of 5 to 50 wt %, preferably 10 to 33 wt % in step i). Examples of such materials are pinolenic oils and concentrates thereof. This process produces a concentrate that contains at least 28 wt% pinolenic acid.

Enzymes suitable for use in steps i), iii), iv) and v) are lipases. Suitable lipases include *Candida rugosa* lipase; Lipase QL; Lipase SL, Lipase OF; *Rhizopus delemar*; lipase; *Rhizopus olyzae* lipase; *Geotrichum candidum* B lipase; and *Rhizomucor miehei* lipase. Preferred enzymes for step i) are *Candida rugosa* lipase and *Geotrichum candidum* B lipase.

Suitable lipases also include Lipozyme IM (a commercial enzyme). The preferred enzyme for use in step iv) is Lipozyme M (from *Rhizomucor miehei*).

The compositions of the invention can be food products. Food products in which pinolenic acid or derivatives thereof or blends containing these compounds can be used include, but are not limited to: margarines; low fat spreads; very low fat spreads; bicontinuous spreads; water continuous spreads; confectionery products, such as chocolates, coatings or fillings; ice creams; ice cream coatings; ice cream inclusions; dressings; mayonnaises; sauces; bakery fats; shortenings; cheese; meal replacement products; health bars; muesli bars; drinks; dairy products; low carbohydrate products; low calorie products; soups; cereals; and milk shakes.

The addition of pinolenic acid or a derivative thereof or blends containing at least one of the compounds to food products can have a positive effect on the texture, hardness, appearance, rheology, oral melt, flavour impact, spreadability, microstructure (crystal size, droplet size), aeration properties or ease of processing of these food products. The use of a glyceride of pinolenic acid is particularly advantageous in this respect.

Other examples of compositions are pharmaceutical compositions, such as in the form of tablets, pills, capsules, chaplets, multiparticulates including: granules, beads, pellets and micro-encapsulated particles; powders, elixirs, syrups, suspensions and solutions. Pharmaceutical compositions will comprise a pharmaceutically acceptable diluent or carrier. Pharmaceutical compositions are preferably adapted for administration parenterally (e.g., orally). Orally administrable compositions may be in solid or liquid form and may take the form of tablets, powders, suspensions and syrups. Optionally, the compositions comprise one or more flavouring and/or colouring agents. Pharmaceutically acceptable carriers suitable for use in such compositions are well known in the art of pharmacy. The compositions of the invention may contain 0.1-99% by weight of pinolenic acid. The compositions of the invention are generally prepared in unit dosage form. Preferably, the unit dosage of pinolenic acid is from 1mg to 1000mg (more preferably from 100mg to 750mg). The excipients used in the preparation of these compositions are the excipients known in the art.

Further examples of product forms for the composition are food supplements (which term includes nutritional products), such as in the form of a soft gel or a hard capsule comprising an encapsulating material selected from the group consisting of gelatin, glycerol, starch, modified starch, starch derivatives such as glucose, sucrose, lactose and fructose. The encapsulating material may optionally contain cross-linking or polymerizing agents, stabilizers, antioxidants, light absorbing agents for protecting light-sensitive fills, preservatives and the like. Preferably, the unit dosage of pinolenic acid in the food supplements is from 1mg to 1000mg (more preferably from 100mg to 750mg). The amount of pine nut oil that is used in a unit dosage form is preferably from 100mg to 2000mg, for example 250mg to 1500mg (e.g, 750mg), for example for taking four times a day.

The compositions of the invention may contain other additives that are well known in the art of food and pharmaceutical products including, but not limited to, flavouring ingredients, colouring agents, sweeteners and emulsifiers.

The following non-limiting examples illustrate the invention and do not limit its scope in any way. In the examples and throughout this specification, all percentages, parts and ratios are by weight unless indicated otherwise.

### Examples

### Example 1

An *in*-v*itro* trial was carried out to measure the effect of various free fatty acids on CCK release from intestinal cells. The study showed the effect of free fatty acids of pine nut oil and thus pinolenic acid as a satiety ingredient (see data below).

### Cell culture

The STC1-1 cell line was cultivated at 37°C in a 5% CO2, 95% air atmosphere in RPMI 1640 supplemented with 5% FCS, 2mM glutamine, 100 U/ml penicillin and 50µM streptomycin. Cells were routinely passaged upon reaching 70-80% confluency by washing the cell layer with PBS and incubating with a solution of trypsin-EDTA. Plating density of 2x10⁶ cells by 75 cm² was used for routine subculture.

### Experimental protocol

24 hours before the experiments, STC1 cells were seeded into 6-well culture plates at 40 - 50% confluency. On the day of the experiment, cells were washed in 1 ml Krebs - Ringer bicarbonate buffer (pH 7,4) containing 0.2% (wt/vol) BSA (KRBB). STC1 cells were pre- incubated for 15 min in 2 ml KRBB, before being incubated in a 2-ml amount of KRBB, with or without the tested agents, for 1 hour. At the end of incubation the supernatant was collected, centrifuged at 1000 rpm for 5 min and immediately frozen at -20°C for RIA. DNA content was measured by fluorometry after extraction of the culture cell contents.

### RIA analysis

CCK immunoreactivity was measured using antiserum .39A (1/300,000) that cross reacts 100% with CCK-33 and CCK-8, 12% with sulfated gastrin-17, 5% with unsulfated gastrin-17, and less than 0.1 % with unsulfated CCK-8 and gastrin-34. CCK-8 is used as standard. The Bolton-Hunter (Thr, Nle)-CCK-9 was labelled with iodine 125 by the chloramine-T method and purified by reverse-phase HPLC. The assay buffer was 0.05M sodium phosphate pH 7.5. Aliquots of 1µl to 200µl of supernatant were tested in duplicate (day 0). The label is added at day 1 and charcoal precipitation is performed 48h later.

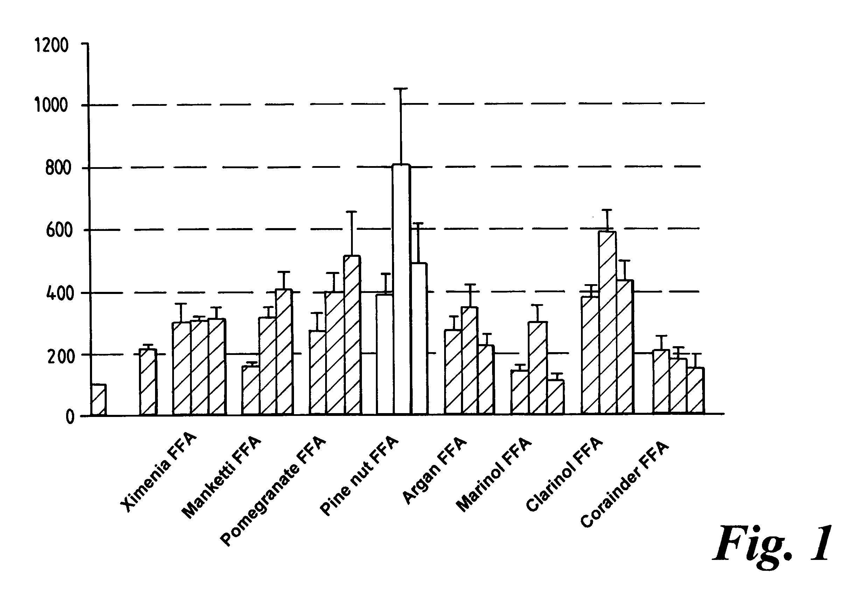

Figure 1 is a bar chart showing CCK levels produced using free fatty acids (FFAs) from pine nuts and other FFAs.

Figure 1 shows that pine nut oil produced a markedly higher level of CCK than the other materials tested. Each sample had CCK level determined at three different concentrations, which were 100 µM, 500 µM and 1mM based on a molecular weight for the FFA of 300 g/mol. Marinol is a fish oil concentrate from Loders Croklaan BV (Wormerveer, The Netherlands) containing EPA and DHA. Clarinol is a product from Loders Croklaan BV (Wormerveer, The Netherlands) containing 80 % conjugated linoleic acid. The other FFA samples are from a range of different natural products.

### Example 2

The following is an example of a filled gelatin capsule according to the invention. A composition comprising pinolenic acid is encapsulated into a gelatin capsule according to methods well-known in the art. The resulting encapsulated product contains 500 mg of pinolenic acid and one capsule can be taken up to four times daily by an adult human.

### Example 3

The following figures of the drawings are referred to in Example 3 and are summarised as follows:
Figure 2 shows CCK blood levels (pmol/L) after intake of 3 g pinolenic acid FFA (upper plot; broken line) or placebo (lower plot; solid line);
Figure 3 shows GLP1 blood levels (pmol/L) after intake of 3 g pinolenic acid FFA (upper plot; broken line) or placebo (lower plot; solid line);
Figure 4 shows the desire to eat during 4 hours after intake of 3 g pinolenic acid FFA (lower plot; broken line) or placebo (upper plot; solid line);
Figure 5 shows the prospective food intake during 4 hours after intake of 3 g pinolenic acid FFA (lower plot; broken line) or placebo (upper plot; solid line);
Figure 6 shows CCK blood levels (pmol/L) after intake of 3 g pinolenic acid Example 3(ii) TG (upper plot; broken line) or placebo (lower plot; solid line);
Figure 7 shows the desire to eat during 4 hours after intake of 3 g pinolenic acid TG (lower plot; broken line) or placebo (upper plot; solid line); and
Figure 8 shows the prospective food intake during 4 hours after intake of 3 g pinolenic acid TG (lower plot; broken line) or placebo (upper plot; solid line).

In the figures, an asterisk indicates a significant difference.

Two compositions comprising pinolenic acid were assayed: Example 3(i) a composition comprising pinolenic acid in the free fatty acid (FFA) form; and Example 3(ii) a composition comprising pinolenic acid in the triglyceride (TG) form. The fatty acid methyl ester (FAME) analysis of the two compositions is as follows:

| **Trivial name** | **Chemical name** | **Abbreviation** | **Crude pine nut oil** | **Example 3(ii) TG** | **Example 3(i) FFA** |
|---|---|---|---|---|---|
| palmitic acid | hexadecanoic acid | C16:0 | 4.3 | 4.1 | 4.4 |
| palmitoleic acid | *cis*-9 hexadecenoic acid | C16:1 | 0.2 | 0.2 | 0.2 |
| margaric acid | heptadecanoic acid | C17:0 | 0.1 | 0.1 | 0.1 |
| stearic acid | octadecanoic acid | C18:0 | 2.1. | 2.1 | 2.1 |
| oleic acid | cis-9 octadecenoic acid | C18:1 | 24.4 | 24.5 | 24.9 |
| linoleic acid | *cis*-9,12 octadecadienoic acid | C18:2 | 44.3 | 45.5 | 45.9 |
| taxoleic acid | *cis*-5,9 octadecadienoic acid | 5,9-C18:2 | 2.1 | 2.2 | 2.1 |
| linolenic acid | *cis*-9,12,15 octadecatrienoic acid | 9,12,15-C18:3 | 0.3 | 0.2 | 0.2 |
| pinolenic acid | *cis*-5,9,12 octadecatrienoic acid | 5,9,12-C18:3 | 15.6 | 15.9 | 15.8 |

A randomized, cross-over, placebo controlled double-blind study was carried out with 18 overweight women (BMI=25-30) receiving 3 g composition of Example 3(i) FFA, 3 g composition Example 3(ii) TG or 3 g placebo (olive oil) capsules in combination with a light low fat breakfast consisting of two slices of white bread and marmalade. At 0, 30, 60, 90, 120, 180 and 240 minutes blood samples were taken for analyses of CCK, GLP-1, glucose, insulin, free fatty acids and triglycerides. Subjective feelings of satiety were evaluated by using visual analogue scales (VAS) for desire to eat, and prospective food consumption. Each subject received the three treatments in a randomized order within a period of two weeks with a wash-out period of one week, according to a Latin square design.

VAS scales consisted of 150-mm horizontal lines with phrases anchored at each end that expressed the most positive or most negative sensation. Subjects drew a vertical line at the point on the horizontal line that corresponded to their hunger sensation. Distances on the VAS scales were converted into scores between 0 and 100.

CCK and GLP-1 concentrations were measured in the blood samples using optimized and validated commercial human RIA kits. Treatments were statistically evaluated with ANOVA and considered significant with a P < 0.05.

Adverse events (AEs) were monitored.

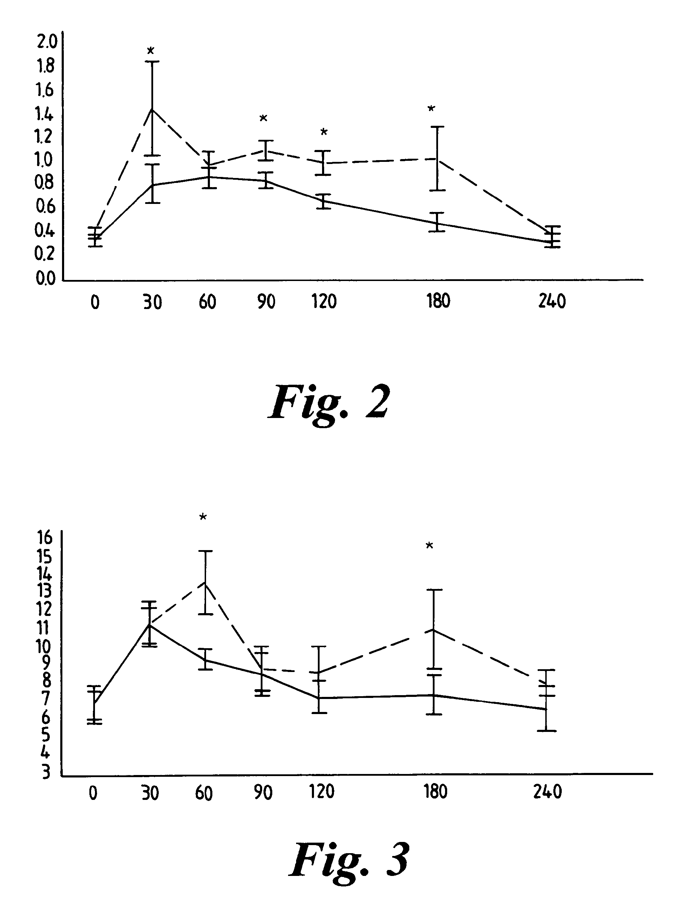

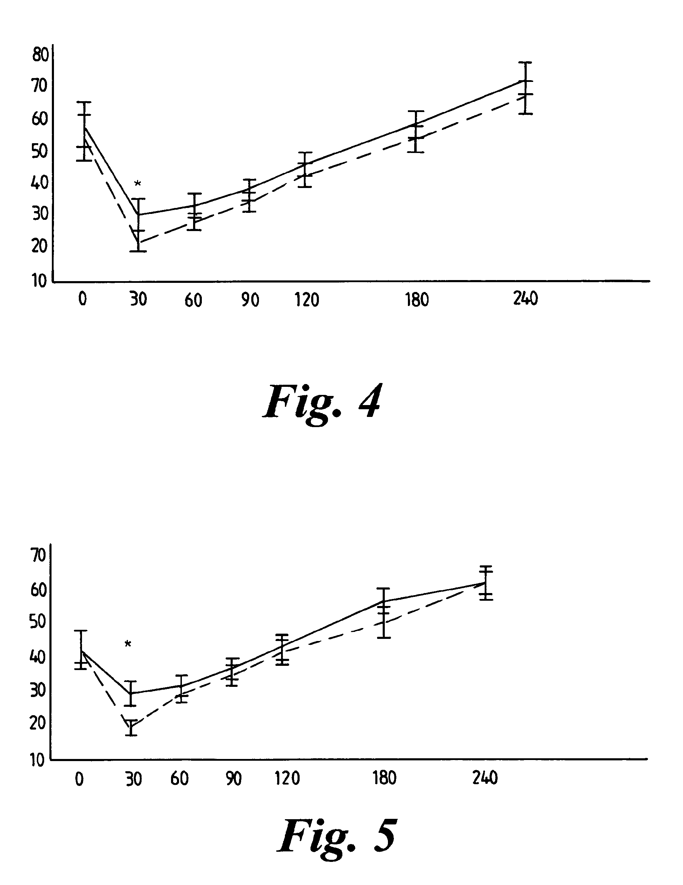

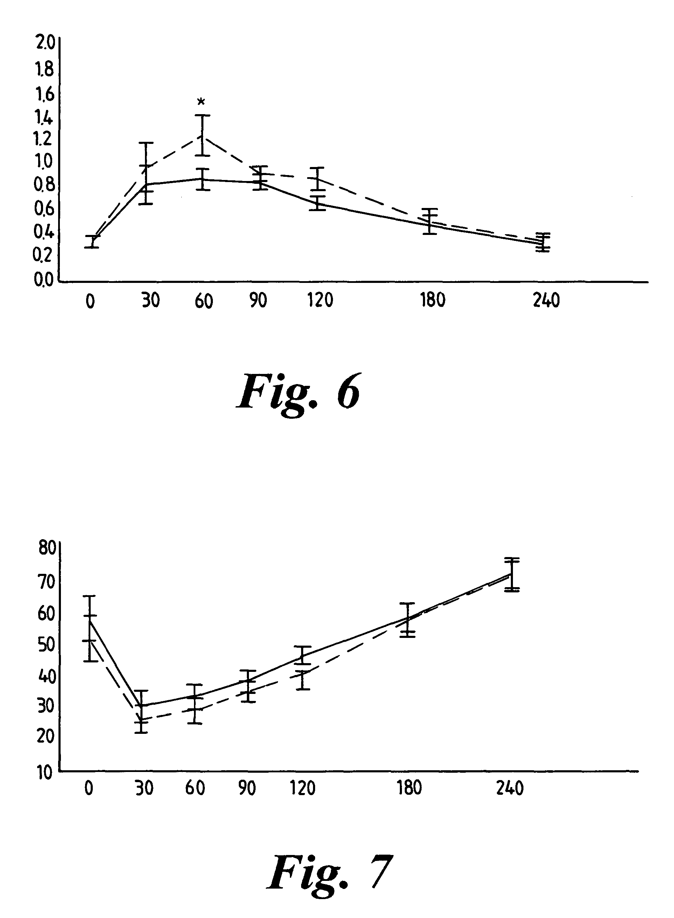

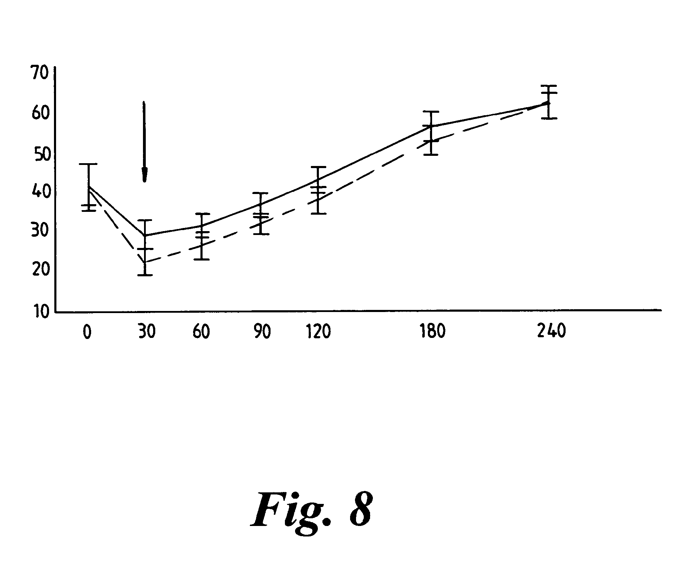

Intake of 3 g of composition Example 3(i) FFA induced at peak in CCK release after 30 minutes which was significantly higher than CCK release in response to placebo (Figure 2). GLP-1 release peaked at 60 minutes after composition of Example 3(i) FFA intake and also was significantly higher than the level of GLP-1 in response to the placebo (Figure 3). Over a period of 4 hours, the total amount of CCK released into the bloodstream in response to composition of Example 3(i) FFA intake was 60% higher and for GLP1 levels were 25% higher than placebo, as measured by the areas under curve. Composition of Example 3(i) FFA also decreased the "desire to cat" and the "prospective intake" scores during the 4 hours after intake (Figures 4 and 5). These scores were lowest at 30 minutes after composition of Example 3(i) FFA and the differences with placebo were significant. Composition of Example 3(ii) TG affected appetite sensations and CCK release similarly to the composition of Example 3(i) FFA (Figures 6 to 8).

In summary, the CCK and GLP1 data showed clear and significant treatment effects. The FFA and TG compositions increased levels of the satiety-inducing hormones, CCK and GLP1, in blood within 30-60 minutes and the levels remained higher for up to four hours after intake. Furthermore, the desire to eat and the prospective intake scores at 30 minutes were less after intake of the Example 3(i) FFA and Example 3(ii) TG compositions than after placebo.

Adverse events or serious adverse events were not reported. Furthermore, the blood levels of glucose, insulin, free fatty acids and triglyceride levels measured from 0-4 hours after intake of the capsules were determined and showed that the triglycerides and free fatty acid levels did not significantly differ at any time point between the groups. Glucose and insulin levels increased in both groups due to the intake of the light sugar-containing breakfast that all participants consumed. In all groups, the glucose and insulin responses during the 4 hours were similar indicating that Example 3(i) FFA and Example 3(ii) TG were well tolerated.

However, the glucose levels did not increase as much after composition of Example 3(i) FFA than after composition Example 3(ii) TG and placebo and more gradually decrease after composition of Example 3(i) FFA and composition of Example 3(ii) TG than after the placebo olive oil. As a result, the insulin level more slowly increased and subsequently quickly decreased after composition of Example 3(i) FFA than after placebo and after composition of Example 3(ii) TG. Thus, composition of Example 3(i) FFA caused more moderate blood level changes in glucose and insulin than placebo with composition of Example 3(ii) TG being in between.

### Example 4

The relative CCK release by a number of different compounds was determined in vitro an *in*-*vitro* trial to measure the effect of various free fatty acids on CCK release from intestinal cells. The study showed the effect of free fatty acids of pine nut oil and thus pinolenic acid as a satiety ingredient (see data below). Other fatty acids present in high and low amounts in pine nut oil (like oleic acid, linoleic acid, taxoleic acid, sciadonic acid and juniperonic acid) are not able to induce high amounts of CCK. A compostion with 27% pinolenic acid induces slightly higher amounts of CCK one with 16 % pinolenic acid.

### Cell culture

The enteroendocrine STC1-1 cell line was cultivated at 37°C in a 5% CO₂, 95% air atmosphere in standard culture medium (DMEM). Cells were routinely passaged upon reaching 70-80% confluency by washing the cell layer with PBS and incubating with a solution of trypsin-EDTA. Plating density of 2x10⁶cells by 75 cm² was used for routine subculture.

### Experimental protocol

STC1 cells were seeded into 6-well culture plates and incubated with control culture medium, with or without the tested agents, for 1 hour. All agents were tested at a 100 µM concentration in the free fatty acid form. Since fatty acids were diluted into ethanol the effect of ethanol was tested and indicated the baseline CCK levels. Capric acid was used as a negative control fatty acid, because it is already known that capric acid does not induce CCK. Effects of bombesin was used as positive control. At the end of incubation the supernatant was collected, centrifuged and immediately frozen at -20°C for RIA.

Cell viability was checked by microscopic analysis, analyzing DNA content which was measured by fluorometry after extraction of the culture cell contents, and in addition LDH release was measured.

### RIA analysis

CCK release was measured using a standard CCK RLA and effects of fatty acids were measured in 6-fold.

The table below shows that the samples with 16% and 27% pinolenic acid respectively produced about a 4-fold and 5-fold higher level of CCK than the negative control fatty acid, capric acid. An oil derived from *Pinus pinea* containing less than 1% pinolenic acid was less well able to induce CCK release (only about 2-fold relative to capric acid). Other fatty acids present in pine nut oil like oleic acid, linoleic acid, taxoleic acid, juniperonic and sciadonic acid were also not able to induce high amounts of CCK (about 2-fold relative to capric acid). In contrast other 18:3 fatty acids like punicic acid, gamma-linolenic, alpha-linoleic, and alpha eleostearic acid are good inducers of CCK similar to pinolenic acid, but it is of note that these fatty acids are measured in a pure form whereas pinolenic acid was only tested at 16% and 27% purity. 95% pure CLA (a 50:50 mix of c9,t11 and t10, c12 isomers) was not able to induce high amounts of CCK similar to CLA with pinolenic acid. A commercial product consisting of a mix from fractioned palm oil and oat oil was also not very active in inducing CCK (2-fold relative to capric acid).

| Compound | Relative amount of CCK released |
|---|---|
| 16% pinolenic acid | 188 |
| 17% pinolenic acid | 210 |
| *Oil from Pinus pinea* (0.35% pinolenic acid) | 101 |
| Oleic acid 18:1 | 90 |
| Linoleic acid 18:2 | 68 |
| Gamma-linolenic acid 18:3 | 140 |
| Alpha-linolenic acid 18:3 | 177 |
| Alpha-eleostearic acid 18:3 | 212 |
| Punicic acid 18:3 | 320 |
| Palm oil /oat oil (commercial product) | 91 |
| CLA mix + 16% pinolenic acid | 112 |
| Capric acid | 43 |
| Ethanol | 53 |

### Example 5

Pinolenic acid TG and pinolenic FFA compositions of Examples 3(i) and 3(ii) are produced by using "crude pine nut oil" as raw material.

Pine nuts are crushed by the suppliers in an expeller by applying high pressure at room temperature. The only heat generated during the expelling is caused by the crushing of the seeds in the expeller, and it never reaches more than 50°C. The extracted oil is then filtered by passing it through canvas. The product obtained is "crude pine nut oil".

The crude oil is further processed to obtain the TG and FFA compositions. Processing consists of the following main steps: refining, hydrolysis and distillation.

The refining step is a physical refining consisting of a bleaching step using bleaching earth in order to remove residues of contaminants, followed by a deodorization step (steam stripping). The refined pine nut oil is the TG composition.

The refined pine nut oil can be further hydrolyzed using a food grade lipase to obtain free fatty acids, glycerol and residues of not fully hydrolyzed oil (di- and tri-glycerides). Finally, the hydrolyzed mixture is distilled to obtain the FFA composition.

### Example 6

Soft gel capsules are produced by rotary die processing. The material for the outside shell of the capsules, the gel, and the fill are formulated separately. Once the gel mass and the fill mass are ready, the gel is spread into thin film to form two gelatin ribbons which are then rolled over two separate dies which determine the size and the shape of the capsules. As the gelatin films adapt to the dies, the fill is carefully dosed to a level of 500mg, 750mg or 1000mg oil per capsule and injected between the two gelatin ribbons which are sealed immediately afterwards by applying heat and pressure. Capsules fall from the machine and are then dried under a stream of hot air.

The following numbered paragraphs are aspects of the invention.
1. Use of pinolenic acid or a derivative thereof in the manufacture of a composition for weight management by reducing the feeling of hunger and/or increasing satiety.
2. Use according to paragraph 1, wherein the composition is in the form of a food supplement, a pharmaceutical composition or a food composition.
3. Use according to paragraph 1 or 2, wherein the composition comprises pinolenic acid as a free fatty acid, a mono-, di- or triglyceride, or a mixture thereof.
4. Use according to any one of paragraphs 1 to 3, wherein the composition is pine nut oil or is derived from pine nut oil.
5. Use as claimed in paragraph 1, wherein the derivative is selected from alpha-linolenic acid, gamma-linolenic acid, punicic acid, eleostearic acid and salts or alkyl esters thereof.
6. Use as claimed in paragraph 5, wherein the alkyl esters are mono-, di- or tri-glycerides or mixtures thereof.
7. Use according to any one of the preceding paragraphs, wherein the composition additionally comprises a fatty acid or derivative thereof selected from the group consisting of linoleic acid, oleic acid, conjugated linoleic acid, enriched isomer mixtures of conjugated linoleic acid, EPA and DHA, or a mixture thereof.
8. Use according to paragraph 7 wherein the fatty acid or derivative thereof is present as a free fatty acid, or a mono-, di- or triglyceride or a mixture thereof.
9. Use according to paragraph 1, wherein the composition further comprises at least one glyceride formed from linoleic acid, oleic acid, trans acids and saturated fatty acids and the composition is a food composition.
10. Use according to paragraph 9, wherein the glyceride is selected from liquid oils selected from soybean oil, sunflower oil, rape seed oil and cotton seed oil; cocoa butter and cocoa butter equivalents; palm oil and fractions thereof; enzymically made fats; fish oils and fractions thereof; conjugated linoleic acid and enriched isomer mixtures thereof; gamma linoleic acid and enriched mixtures thereof; hardened liquid oils; and mixtures thereof.
11. Use according to any of the preceding paragraphs, wherein the composition is a food product selected from the group consisting of: margarines; low fat spreads; very low fat spreads; bicontinuous spreads; water continuous spreads; confectionary products, such as chocolates, coatings or fillings; ice creams; ice cream coatings; ice cream inclusions; dressings; mayonnaises; sauces; bakery fats; shortenings or cheese; meal replacement products; health bars; muesli bars; drinks; dairy products; low carbohydrate products; low calorie products; soups; cereals and milk shakes.
12. Use according to any one of paragraphs 1 to 8, wherein the composition is a pharmaceutical composition in a form selected from the group consisting of tablets, pills, capsules, caplets, multiparticulates including: granules, beads, pellets and micro-encapsulated particles; powders, elixirs, syrups, suspensions and solutions.
13. Use according to any one of paragraphs 1 to 8, wherein the composition is a food supplement in the form of a soft gel or a hard capsule comprising an encapsulating material selected from the group consisting of gelatin, starch, modified starch, and starch derivatives.
14. Use according to any one of the preceding paragraphs, wherein the composition is for weight management by helping to maintain body weight and/or to reduce body weight and/or assisting in slimming the body and/or reducing calorie intake and/or allowing less room for high calorie foods.
15. Use as claimed in any one of paragraphs 1 to 14, wherein the mammal is a human.
16. Use of a composition as defined in any one of paragraphs 1 to 14, in the manufacture of a composition for stimulating the production of cholecystokinin (CKK) in a mammal.

## Claims

1. Use of pinolenic acid or a derivative thereof in the manufacture of a composition for weight management by reducing the feeling of hunger and/or increasing satiety, wherein the composition is in the form of a pharmaceutical composition.

2. Use according to Claim 1, wherein the composition comprises pinolenic acid as a free fatty acid, a mono-, di- or triglyceride, or a mixture thereof.

3. Use according to Claim 1 or Claim 2, wherein the composition is pine nut oil or is derived from pine nut oil.

4. Use as claimed in Claim 1, wherein the derivative is selected from alpha-linolenic acid, gamma-linolenic acid, punicic acid, eleostearic acid and salts or alkyl esters thereof.

5. Use as claimed in Claim 4, wherein the alkyl esters are mono-, di- or tri-glycerides or mixtures thereof.

6. Use according to any one of the preceding claims, wherein the composition additionally comprises a fatty acid or derivative thereof selected from the group consisting of linoleic acid, oleic acid, conjugated linoleic acid, enriched isomer mixtures of conjugated linoleic acid, EPA and DHA, or a mixture thereof.

7. Use according to Claim 6 wherein the fatty acid or derivative thereof is present as a free fatty acid, or a mono-, di- or triglyceride or a mixture thereof.

8. Use according to any one of Claims 1 to 7, wherein the composition is in a form selected from the group consisting of tablets, pills, capsules, caplets, multiparticulates including: granules, beads, pellets and micro-encapsulated particles; powders, elixirs, syrups, suspensions and solutions.

9. Use according to any one of the preceding claims, wherein the composition is for weight management by helping to maintain body weight and/or to reduce body weight and/or assisting in slimming the body and/or reducing calorie intake and/or allowing less room for high calorie foods.

10. Use as claimed in any one of Claims 1 to 9, wherein the mammal is a human.

11. Use of a composition as defined in any one of Claims 1 to 10, in the manufacture of a composition for stimulating the production of cholecystokinin (CKK) in a mammal.
